# EUROPÄISCHE PATENTANMELDUNG

(11) **EP 0 639 355 A1**
(43) Veröffentlichungstag der Anmeldung: **22.02.1995**
(21) Anmeldenummer: 94110313.7
(22) Anmeldetag: 02.07.1994
(51) Int. Cl.: A61F 2/00

(54) **Prothese zur Verhinderung der Harninkontinenz bei Frauen**

(30) Priorität: 21.08.1993 DE 4328158
(71) Anmelder: RULL, Johann, Dr. med., D-71522 Backnang (DE)
(72) Erfinder: RULL, Johann, Dr. med., D-71522 Backnang (DE)

(57) **Zusammenfassung**

Die Erfindung betrifft eine Prothese zur Verhinderung der Harninkontinenz bei Frauen durch Korrektur des vesico-urethralen Winkels mit einer als einstellbarer Abstandhalter zwischen Vagina und Harnröhrenansatz einsetzbaren, mit Fluid gefüllten bzw. füllbaren und in ihrer Höhe verstellbaren Elevationskammer (1), deren obere Außenfläche (7) als nicht einengende Auflagefläche für die Harnröhre (9) und deren untere Außenfläche (8) als Abstützfläche zum Aufsetzen auf die Oberseite der Vagina ausgebildet sind und deren den Abstand bestimmende Höhe in implantierten Zustand durch Punktion und Korrektur der Fluidmenge einstellbar ist. Erfindungsgemäß weist die Prothese mindestens zwei an der Elevationskammer (1) angeordnete flexible Bänder (2, 3) zur losen Fixierung ihrer Lage im Körper auf.

## Beschreibung

Die Erfindung betrifft eine Prothese zur Verhinderung der Harninkontinenz bei Frauen durch Korrektur des vesico-urethralen Winkels mit einer als einstellbarer Abstandhalter zwischen Vagina und Harnröhrenansatz einsetzbaren, mit Fluid gefüllten bzw. füllbaren, in ihrer Höhe verstellbaren Elevationskammer, deren obere Außenfläche als nicht einengende elastische Auflagefläche für die Harnröhre und deren untere Außenfläche als Abstützfläche zum Aufsetzen auf die Oberseite der Vagina ausgebildet sind und deren den Abstand bestimmende Höhe in implantiertem Zustand durch Punktion und Korrektur der Fluidmenge einstellbar ist.

Eine altersbedingte oder krankhafte Blasensenkung bei Frauen führt zu einer Senkung des Harnröhrenansatzes im Bereich zwischen Harnröhre und Blase, verbunden mit einer Vergrößerung bzw. Abflachung des vesico-urethralen Winkels. Dies hat eine Harninkontinenz bei Streßsituationen, zum Beispiel beim Husten, zur Folge. Zur Verhinderung einer solchen Streßinkontinenz ist es erforderlich, den Harnröhrenansatz wieder zu heben, um die natürliche Winkelstellung zwischen Harnröhre und Blase wieder herzustellen.

Hierzu gibt es sogenannte Zügelplastiken, bei denen man die Harnröhre im Bereich des Harnröhrenansatzes mit Faszien untergreift, welche in einem höher gelegenen Bereich des Bauchraumes befestigt werden. Derartige Zügelplastiken sind bei einer falschen Bemessung der Faszienlänge bzw. bei nachträglichen Veränderungen der Lage der Blase nur schwer korrigierbar.

Die GB-A-21 89 999 beschreibt eine Inkontinenzprothese in Form eines halbmondförmigen Hydrogels mit einem Wassergehalt von ca. 90 Gew.%. Diese Prothese wird unterhalb der Harnröhre angeordnet und unter Anhebung der Harnröhre an benachbartes Gewebe angenäht. Auch hier sind spätere Korrekturen nicht oder nur mit operativem Aufwand möglich.

In der EP 0 264 258 A2 ist ein intravaginaler Einsatz beschrieben, der den Harnröhrenansatz zusammen mit der Wandung der Vagina nach oben hebt. Dieser Einsatz kann mit einem aufblasbaren Körper kombiniert sein, der zum Verschließen der Harnröhre aufgeblasen und zum Entleeren der Harnblase abgelassen wird.

Es gibt auch sogenannte Ballonpressen, die um die Harnröhre herumgelegt werden und einen künstlichen Schließmuskel darstellen. Zur Entleerung der Harnblase muß im Einzelfall der Druck abgelassen und danach wieder erhöht werden.

Die WO-91/00069 beschreibt eine Harninkontinenzprothese, die als Abstandhalter zwischen der Oberseite der Vagina und dem Harnröhrenansatz eingesetzt wird. Diese Prothese dient dazu, den natürlichen vesico-urathralen Winkel wieder herzustellen. Die Prothese besteht aus einem mit einer Flüssigkeit gefüllten Hohlkörper, der punktierbar ist. Der richtige Abstand zur Wiederherstellung des Winkels wird einmal eingestellt, gegebenenfalls nachkorrigiert, und braucht dann im Normalfall nicht mehr verändert zu werden. Der Erfindung liegt die Aufgabe zugrunde, diese Harninkontinenzprothese zu verbessern.

Die Erfindung ist dadurch gekennzeichnet, daß die Prothese mindestens zwei an der als Abstandhalter dienenden Elevationskammer angeordnete flexible Bänder zur spannungsfreien Fixierung der Lage der Prothese im Körper aufweist. In der Regel sind vorzugsweise nur zwei Bänder vorgesehen. Die Bänder sind verhältnismäßig langgestreckte Verbindungsmittel, die dazu eingerichtet sind, die Prothese locker in ihrer Position zu halten, ohne Spannungen auf die Prothese oder die Harnröhre auszuüben. Die Verbindungsmittel bzw. Bänder können einen flachen, ovalen oder runden Querschnitt haben und sind vorzugsweise hohl ausgebildet. Die Bänder können bei der Implantation der Prothese an geeigneten Stellen im Körper fixiert, beispielsweise angenäht werden. Sie verhindern dadurch, daß die Prothese bei heftigen Bewegungen im Bauchraum, wie sie bei starkem Husten vorkommen können, aus ihrer vorbestimmten Lage verrutscht und dadurch, zumindest vorübergehend, ihre Funktion nicht mehr ausüben kann. Die Bänder unterscheiden sich aber wesentlich von den sogenannten Zügeln, deren Funktion es ist, den Harnröhrenansatz durch ständigen Zug nach oben hochzuhalten. Demgegenüber stützt sich die Elevationskammer der erfindungsgemäßen Prothese auf der oberen Wandung der Vagina ab. Die Bänder haben lediglich die Funktion, die Elevationskammer in ihrer für die Abstützung richtigen Lage zu halten. Die Bänder sind vorzugsweise länger ausgebildet als erforderlich und können bei der Implantation auf die richtige Länge verkürzt werden. Dies erlaubt es, die Fixierungsstellen für die Bänder den jeweiligen anatomischen Verhältnissen entsprechend auszuwählen und den Größenverhältnissen der Patientin anzupassen. Die Elevationskammer besteht mit Vorteil aus gummielastischem Material, insbesondere aus Silikonkautschuk. Die Fixierungsbänder bestehen mit Vorteil aus dem gleichen Material und sind vorzugsweise einstückig mit der Elevationskammer ausgebildet. Die Bänder sind dadurch längselastisch, was bei besonderen Streßsituationen von Vorteil ist.

An der Prothese ist bei einer bevorzugten Ausführungsform mindestens eine Punktionsstelle vorgesehen, die außermittig an der Elevationskammer angeordnet ist, vorzugsweise Teil einer Punktionskammer ist. Die außermittige Anordnung ermöglicht eine erleichterte Punktion und verhindert selbst bei unsachgemäßer Durchführung der Punktion eine Verletzung der Harnröhre. Mit Vorteil sind mehrere, insbesondere verschiedene Punktionsstellen, insbesondere Punktionskammern, vorgesehen, von denen vorzugsweise mindestens eine an der Elevationskammer angeordnet ist. Es kann mindestens eine Punktionskammer als seitliche Nebenkammer an der Elevationskammer ausgebildet sein. Dabei ist es von besonderem Vorteil, wenn Punktionsstellen von Punktionskammern, die im örtlichen Bereich der Elevationskammer vorgesehen sind, eine Einstichrichtung vorgeben, die von der Lage der Harnröhre abweist. Diese Maßnahme bietet, wie auch die seitliche Anordnung der Punktionskammer, einen Schutz der Harnröhre und auch einen Schutz des Elevationsteils der Prothese.

Die Elevationskammer kann auch mindestens eine mit ihr kommunizierende, räumlich getrennte Teilkammer aufweisen, die als Punktionskammer ausgebildet ist. Eine solche räumlich getrennte Punktionskammer bietet völlige räumliche Unabhängigkeit zwischen Punktierungsstelle und Abstandhalter. Eine solche Punktionskammer kann an für die Punktierung geeigneten Stellen des Körpers implantierbar ausgebildet sein und über eine flexible Leitung mit der Elevationskammer verbunden werden bzw. verbunden sein. So ist eine Punktierung durch die Bauchhaut hindurch bevorzugt und erleichtert auch ein steriles Arbeiten. Eine solche externe Punktionskammer kann auch zur Befestigung mindestens eines Fixierbandes dienen und dementsprechend ausgebildet sein. Dadurch entfällt mindestens eine zusätzliche Befestigung eines Fixierbandes am Körper. Die Punktionskammer kann mit entsprechenden Einrichtungen versehen sein, die ein einfaches Befestigen des Fixierbandes ermöglichen. So kann an eine Punktionskammer ein Fixierband angeformt sein, das mit einem Fixierband der Elevationskammer verbindbar ist. In ähnlicher Weise kann auch die Verbindungsleitung für das Fluid der Prothese, das in der Regel eine Flüssigkeit ist, in der Länge einstellbar ausgebildet sein. Mit Vorteil weist die Punktionskammer einen Leitungsanschluß auf oder einen Leitungsabschnitt, der nach der Implantation der Punktionskammer freiliegt und mit einem Leitungsabschnitt der Elevationskammer verbindbar ist, beispielsweise mittels eines Kupplungsstücks. Falls erwünscht, können im Bereich der Verbindung Verriegelungseinrichtungen vorgesehen sein, die ein Lösen der Verbindung, auch bei in ungünstigen Fällen auftretenden Zugspannungen, verhindern. Vorzugsweise ist die externe Punktionskammer unabhängig von Fixierbändern vorgesehen und ausgebildet.

Bei einer besonders bevorzugten Ausführungsform der Erfindung ist die Elevationskammer so ausgebildet, daß sich zur Veränderung des Abstandes des variablen Abstandhalters im wesentlichen nur die obere Auflagefläche für die Harnröhre in ihrer Höhe verändert. Dies kann dadurch erreicht werden, daß der die obere Auflagefläche bildende Wandungsabschnitt der Elevationskammer aus, im Verhältnis zu den anderen Wandungsbereichen, dünnem weichelastischem Material besteht und leicht dehnbar ist. Bei Änderung des Füllgrades der Elevationskammer hebt und senkt sich dann nur diese obere Auflagefläche, wogegen die übrigen Teile der Elevationskammer und damit die Gestalt der Elevationskammer im wesentlichen unverändert bleiben. Eine nachträgliche Korrektur des Füllgrades wirkt sich dadurch nur auf die Höhe des Abstandes und nicht auf die übrigen Teile der Prothese und benachbarten Körperteile aus. Dementsprechend ist die untere, auf die Vagina aufsetzbare Abstützfläche im Vergleich zur dünnen oberen Auflagefläche von einem verhältnismäßig dickwandigen Wandungsabschnitt der Kammer gebildet, der im wesentlichen nicht dehnbar ist. Er kann zusätzlich noch durch entsprechende Einlagen verstärkt sein. Entsprechendes gilt mit Vorteil auch für die übrigen Oberflächenabschnitte der Elevationskammer und Punktionskammer.

Wie bereits erwähnt, ist die erfindungsgemäße Prothese so ausgebildet, daß die Harnröhre bei ihrer Abstützung nicht zusammengedrückt oder gequetscht wird. Auch ist eine Linien- oder Flächenberührung zwischen Harnröhre und Prothese im Bereich des Harnröhrenansatzes bevorzugt. Mit Vorteil ist deshalb die Auflagefläche für die Harnröhre, d.h. die Auflagefläche des Elevationsteils der Prothese, in der quer zur Harnröhre verlaufenden Richtung konkav und in Richtung der Harnröhre vorzugsweise konvex ausgebildet, wobei die Krümmungsradien jeweils vorzugsweise groß im Vergleich zum Radius der Harnröhre sind.

Der erfindungsgemäßen Prothese kann mit Vorteil eine Stützeinrichtung, insbesondere eine Stützplatte, für die Harnblase zugeordnet sein. Eine solche Stützplatte ist mit Vorteil oberhalb der Elevationskammer, insbesondere oberhalb der Harnröhre, angeordnet und dient dazu, einem Verdrehen der Blase bei besonderen Streßsituationen entgegenzuwirken. Die Stützeinrichtung kann eben ausgebildet oder mit Vorteil auch der Form der Blase angepaßt sein. Vorzugsweise ist die Stützeinrichtung so ausgebildet, daß sie in ihrer Lage durch die Fixierbänder festlegbar ist. Hierzu kann die Stützeinrichtung Durchbrechungen oder Kanäle aufweisen, durch die die Fixierbänder bei der Implantation hindurchführbar sind und die Stützeinrichtung in ihrer senkrechten Lage vor der Blase halten.

Die erfindungsgemäße Prothese ist durch die Bauchwand hindurch im Bereich des Ansatzes der Harnröhre an der Blase implantierbar. Bei einer Kontrolluntersuchung kann nach der Operation geprüft werden, ob der vesico-urethrale Winkel richtig eingestellt ist. Sind Korrekturen erforderlich, dann kann dies durch Punktion an geeigneter Stelle erfolgen. Diese Einstellung entspricht dann den normalen anatomischen Verhältnissen. Nach jeweils größeren Zeitabschnitten kann dann, wenn sich der vesico-urethrale Winkel verändert haben sollte, eine Nachkorrektur erfolgen, ohne daß ein operativer Eingriff erforderlich ist.

Es kann aber vorkommen, daß sich die automatischen Verhältnisse so stark ändern, daß eine Korrektur der Länge der Fixierbänder, insbesondere eine Verlängerung derselben, oder eine Tieferlegung der Fixierstellen am Körper erforderlich wird. Auch ist nicht auszuschließen, daß ein Chirurg versehentlich die Bänder zu kurz einstellt oder die Fixationsstellen am Körper zu hoch ansetzt. In solchen Fällen ist ein operativer Eingriff zur Absenkung der Prothese auf ihre Normallage erforderlich. Um diesen auf möglichst engem Raum begrenzen zu können, sind Korrektureinrichtungen als Führung und Widerlager für Korrekturhilfen vorgesehen, die die Absenkung der Elevationskammer erleichtern. So sind die Fixierbänder bei einer Ausführungsform der Erfindung hohl bzw. rohrförmig ausgebildet, ohne daß ihr Innenraum mit dem Innenraum der Elevationskammer kommuniziert. Vielmehr endet der Hohlraum der Fixierbänder im Bereich der Elevationskammer. Es ist auch möglich, den Fixierbändern bis in den Bereich der Punktionskammer reichende Korrektureinrichtungen, insbesondere flexible Führungsrohre, zuzuordnen, die im Bereich der Elevationskammer ein Widerlager für eine Korrekturhilfe aufweisen, insbesondere verschlossen sind. Als Korrekturhilfe kann dann ein insbesondere flexibler Draht o.dgl. verwendet werden, um die Elevationskammer von der Operationsstelle aus nach unten zu drücken bzw. zu schieben, insbesondere indem der Draht von oben in den Hohlraum der Fixierbänder bzw. der Korrekturrohre eingeschoben wird. Nach Erreichen des unteren Widerlagers kann die Punktionskammer mit dem Draht nach unten geschoben werden. Der Draht wird danach wieder herausgezogen, so daß eine erneute Fixierung der Bänder erfolgen kann.

Weitere Merkmale der Erfindung ergeben sich aus der nachfolgenden Beschreibung von bevorzugten Ausführunsgformen in Verbindung mit der Zeichnung und den Unteransprüchen. Hierbei können die einzelnen Merkmale jeweils für sich allein oder zu mehreren in Kombination miteinander bei einer Ausführungsform der Erfindung verwirklicht sein. In der Zeichnung zeigen:
Fig. 1 eine Ausführungsform der Erfindung, teilweise geschnitten,
Fig. 2 einen Querschnitt durch die Ausführungsform nach Fig. 1 entlang der Linie A-A,
Fig. 3 eine andere Ausführunsgform der Erfindung,
Fig. 4 eine weitere Ausführunsgform der Erfindung, und
Fig. 5 eine schematische Darstellung einer mit einer Stützplatte für die Blase ausgerüstete Ausführungsform.

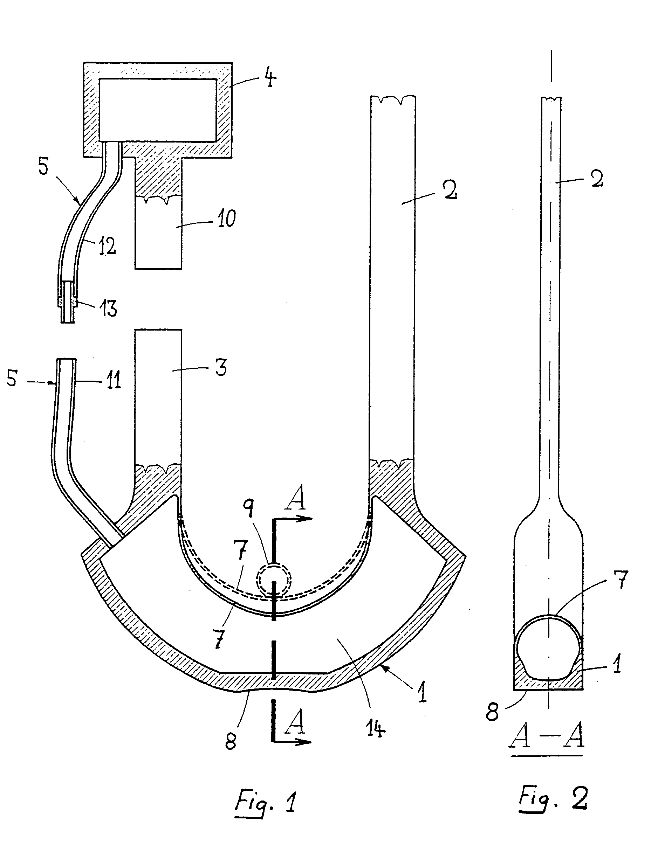

Die in den Fig. 1 und 2 dargestellte Prothese weist eine Elevationskammer 1, zwei daran angeformte Bänder 2 und 3 sowie eine externe Punktionskammer 4 auf, die mit der Elevationskammer 1 über eine Flüssigkeitsleitung 5 kommunizierend verbunden ist. Die externe Punktionskammer kann im Abstand von der Elevationskammer unter der Bauchhaut oder total implantiert werden. Im wesentlichen die gesamte Prothese besteht aus Silikonkautschuk, wobei unterschiedliche mechanische Eigenschaften einzelner Stellen der Prothese durch unterschiedliche Wandstärken bedingt sind.

Die Elevationskammer 1 besteht aus einem im wesentlichen U-förmig verlaufenden, bogenförmigen Schlauchabschnitt, der einen geschlossenen Hohlkörper bildet. Die Außenwandungen 6 der Elevationskammer sind relativ dickwandig und zwar flexibel, aber im wesentlichen nicht dehnbar. Demgegenüber ist die die Innenseite des Bogens bildende Wandung 7 an der Oberseite der Elevationskammer dünnwandig ausgebildet und dehnbar. An ihrer Unterseite ist die Elevationskammer 1 abgeflacht und bildet eine Abstützfläche 8, die sogar leicht konkav verlaufen kann. Die Abstützfläche dient bei der Implantation der Prothese zur Auflage auf der Oberseite der Vagina, um den Abstand zwischen Vagina und Harnröhre 9 einstellen zu können. Die Harnröhre 9 ruht nach Implantation der Prothese auf der als Auflagefläche dienenden dehnbaren Oberseite 7 (Elevationsteil) der Elevationskammer, also auf einer weichelastischen Grundlage. Wie aus Fig. 1 zu ersehen ist, ist der Durchmesser an der inneren Oberseite 7 des Bogens der Elevationskammer um ein Vielfaches größer als der Durchmesser der Harnröhre, so daß diese auf der Oberseite 7 ohne Einengung zu liegen vermag. Die Elevationskammer 1 ist zusammen mit ihrer Punktionskammer 4 und der Verbindungsleitung 5 mit einer Flüssigkeit vollständig befüllbar. Die Befüllung erfolgt bei der Implantation. Durch Einstellen des Füllgrades kann der Abstand zwischen Vagina und Harnröhre 9 eingestellt und damit der Winkel zwischen Blasenansatz und Harnröhre auf das richtige Maß korrigiert werden. In Fig. 1 zeigt die gestrichelte Darstellung der Wandung 7 an der Oberseite der Elevationskammer 1 einen teilweise erhöhten Zustand und damit vergrößerten Abstand.

Die Fixierbänder 2 und 3 sind an den beiden Enden der bogenförmigen Elevationskammer 1 angeformt und sind als Bänder aus Vollmaterial ausgebildet. Die Bänder dienen dazu, die Prothese im implantierten Zustand locker in ihrer Lage zu fixieren, um zu vermeiden, daß sie bei starken Pressungen im Bauchraum, beispielsweise beim Husten, aus ihrer Lage verschoben wird. Die Fixierung mittels der Bänder hat jedoch keinen Einfluß auf die Funktion der Elevationskammer 1 als Abstandhalter. Sie üben im Normalzustand keine Zugwirkung in Richtung nach oben aus. Die Länge des Fixierbandes 2 ist größer als erforderlich. Das obere Ende des Bandes wird nach entsprechender Kürzung bei der Implantation an einer geeigneten Stelle im Körper, beispielsweise suprapubisch, an einer Faszie/Symphyse angenäht.

Die Punktionskammer 4 ist ebenfalls suprapubisch implantierbar und wird so unter der Bauchhaut implantiert, daß sie durch die Bauchhaut hindurch punktierbar ist. Gleichzeitig ist die Punktionskammer als Fixationselement für die Elevationskammer 1 ausgebildet. Sie weist hierzu ein angeformtes Fixierband 10 auf, dessen freies Ende mit dem freien Ende des an der Elevationskammer angeformten Fixierbandes 3 nach entsprechender Verkürzung auf die richtige Länge verbindbar ist, was beispielsweise durch Vernähen erfolgen kann. In ähnlicher Weise wie das zweite Fixierband 3, 10 zweiteilig ausgebildet ist, setzt sich auch die Verbindungsleitung 5 aus zwei Teilen 11 und 12 zusammen, die über ein Kupplungsstück 13 zur Herstellung der Flüssigkeitsverbindung zwischen Punktionskammer 4 und Elevationskammer 1 miteinander verbindbar sind. Auch die Leitungsabschnitte 11 und 12 sind länger als erforderlich und können beim Implantieren der Prothese auf das gewünschte Maß gekürzt werden. Dabei wird mit Vorteil die Leitung 5 insgesamt länger gehalten als das Fixierband 3, 10, um die Leitung auch bei Streßsituationen spannungsfrei zu halten.

Es ist mit Vorteil auch möglich, die Punktionskammer 4 unabhängig von Fixierbändern vorzusehen, so daß für die Implantation der Punktionskammer und für die Fixierung der Bänder getrennte Körperstellen auswählbar sind.

Die Prothese kann durch den Bauchraum implantiert und wie erwähnt fixiert werden. Während der Implantation werden der Hohlraum 14 der Elevationskammer 1 und die Hohlräume der Verbindungsleitung 5 und der Punktionskammer 4 mit Flüssigkeit gefüllt, wobei der Füllgrad so gewählt wird, daß der gewünschte Abstand zwischen Vagina und Harnröhre 9 erreicht wird. Zeigt sich nach abgeschlossener Operation, daß der Abstand zu hoch gewählt ist, so daß es zur Zurückhaltung von Restharn in der Blase kommt, oder daß er zu gering ist, so daß der Harnröhrenansatz mit der Blase noch nicht den gewünschten Winkel bildet, dann kann der Abstand nachträglich durch Entnahme oder weitere Zufuhr von Flüssigkeit korrigiert werden. Dies ist auch möglich, wenn sich die anatomischen Verhältnisse mit der Zeit ändern.

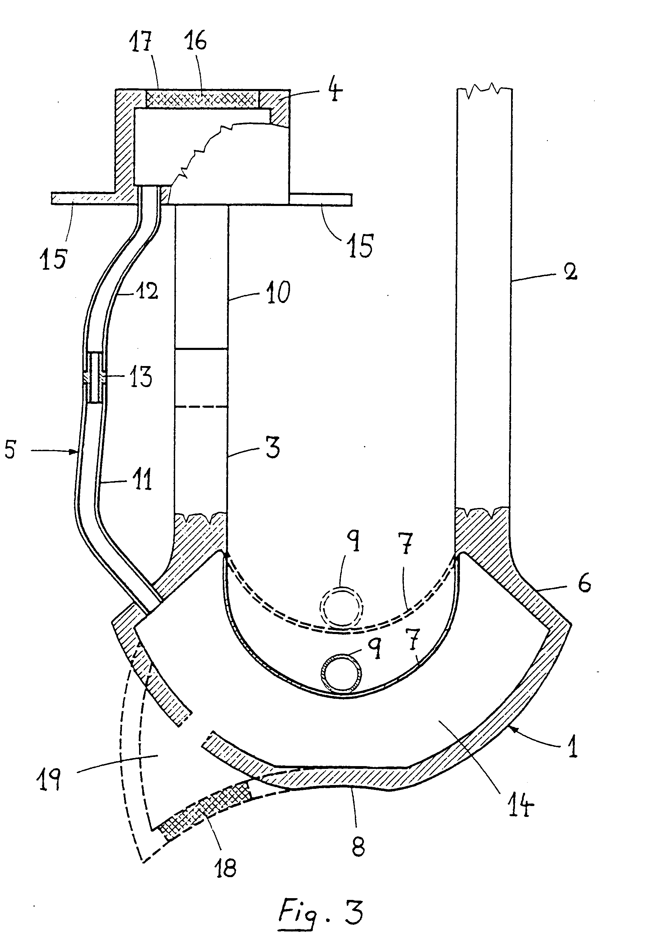

Die in Fig. 3 dargestellte Ausführungsform der Erfindung ist im Prinzip ähnlich aufgebaut wie die Ausführungsform nach Fig. 1. Deshalb werden für entsprechende Teile die gleichen Bezugsziffern verwendet. In Fig. 3 ist die Prothese in einem Zustand dargestellt, in dem sowohl die mechanische Verbindung zwischen der Punktionskammer 4 und der Elevationskammer 1 als auch die Flüssigkeitsverbindung über die Leitung 5 fertiggestellt sind. Die Punktionskammer 4 weist zusätzlich einen flanschförmigen Rand 15 auf, der zum Annähen der Punktionskammer an benachbartes Gewebe dient. Die Punktionskammer weist weiterhin eine Verstärkung 16 in ihrer Oberseite 17 auf, die nach Implantation als Punktierungsstelle dient. Diese Verstärkung, die eine Einlage sein kann, oder auch eine oder mehrere ringförmige Einfassungen aufweisen kann, innerhalb derer das Wandungsmaterial verdichtet ist, erhöht die Widerstandskraft der Punktionsstelle und erlaubt ein mehrfaches Punktieren ohne Beeinträchtigung der Dichtigkeit.

Die Ausführungsform nach Fig. 3 weist eine falkultative zweite Punktionsstelle 18 auf, die in Fig. 3 gestrichelt dargestellt ist. Diese Punktionsstelle 18 ist Teil einer zweiten Punktionskammer 19, die seitlich an die Elevationskammer 1 angeformt ist. Diese Punktionskammer 19 kann alternativ zur Punktionskammer 4 zu Korrekturen durch Punktieren herangezogen werden. Die Punktionsstelle 18 ist durch die obere Wandung der Vagina hindurch mit einer Nadel erreichbar. Der die Punktierungsstelle 18 bildende Wandungsabschnitt der Punktierungskammer 19 ist dabei aus der Wandung der Elevationskammer 1 so abgewinkelt, daß die senkrecht zur Punktierungsstelle 18 verlaufende Einstichrichtung von der Harnröhre 9 abweist, so daß keine Gefahr einer eventuellen Verletzung der Harnröhre und des Elevationsteils der Punktionskammer bei unsachgemäßem Punktieren besteht. Es können auch zwei seitliche Punktionskammern vorgesehen sein, die spiegelbildlich angeordnet sind. Die Unterseite der Elevationskammer kann konkav ausgebildet sein, beispielsweise unter Einbeziehung von zwei seitlichen Punktionskammern, so daß eine seitliche Stabilisierung der Lage auf der Vagina erhalten wird.

Fig. 3 zeigt wiederum zwei verschiedene Füllgrade der Elevationskammer 1 mit entsprechender Anhebung der Oberseite 7 und der Harnröhre 9. Die Abstände liegen weiter auseinander als in Fig. 1 gezeigt. Bei der erfindungsgemäßen Prothese kann der Abstand zwischen einem minimalen Füllgrad und einem maximalen Füllgrad um mindestens das Zweifache vergrößert werden.

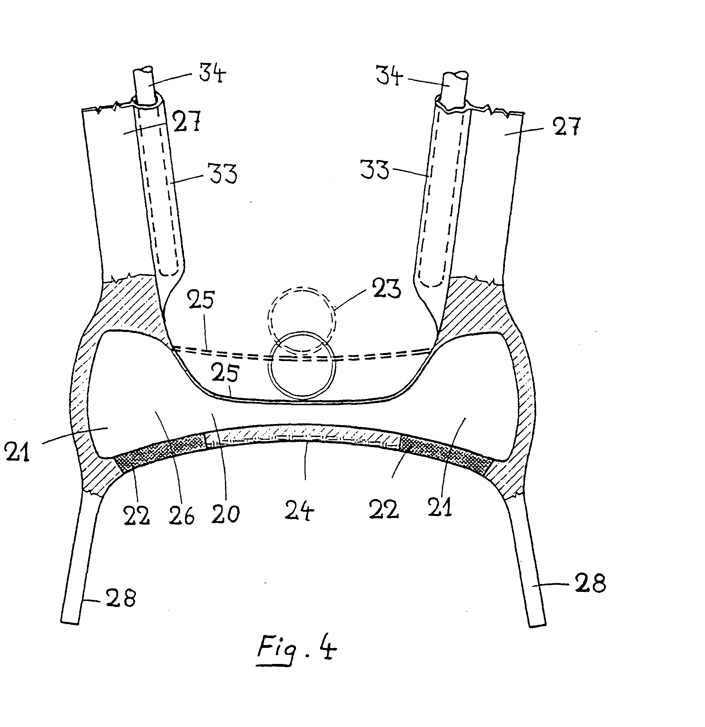

Die in Fig. 4 dargestellte Ausführungsform der Erfindung besitzt eine Elevationskammer 20 mit zwei seitlichen, in die Elevationskammer integrierten vaginalen Punktionskammern 21. Als Punktionsstellen dienende Flächenabschnitte 22 der Punktionskammern 21 bzw. Elevationskammer 20 sind seitlich nach außen abfallend geneigt, wodurch wiederum eine Einstichrichtung gebildet wird, die von der Harnröhre 23 abweist. Dadurch erhält die Unterseite 24 der Elevationskammer 20 eine konkave Form, die zusammen mit der konkaven Ausbildung der dünnwandigen Oberseite 25 der Elevationskammer eine Verjüngung der Elevationskammer 20 im mittleren Bereich ergibt, die insbesondere bei niedrigem Füllgrad der Kammer stark ausgeprägt ist. Im Bereich der seitlichen Punktionskammern 21 hat der Hohlraum 26 der Elevationskammer 20 im Vergleich zur Mitte erheblich größere Ausmaße, unter anderem um eine ausreichende Einstichtiefe bei der Punktion zu gestatten.

Die Ausführungsform nach Fig. 4 ist H-förmig ausgebildet und weist vier Ansätze auf, von denen zwei obere Fixierbänder 27 an der Oberseite der Punktionskammern aus der Elevationskammer herausgeformt sind und zwei nach unten ragende, kurz ausgebildete seitliche Ansätze 28 an der Außenseite der Punktionsstellen 22 der Punktionskammern 21 angeformt sind und eine zusätzliche seitliche Stabilisierung der Prothese von unten ermöglichen. Die nach unten ragenden Ansätze 28 werden normalerweise nicht am Körper fixiert.

Fig. 4 zeigt wiederum die Möglichkeit, die Harnröhre 23 allein durch den unterschiedlichen Füllgrad des Hohlraumes 26 der Elevationskammer 20 anzuheben, um dadurch den richtigen Winkel zwischen Harnblase und Harnröhrenansatz herzustellen.

Parallel zu den Fixierbändern 27 angeordnet und mit diesen verbunden sind flexible Führungsrohre 33, die nach oben bis in den Bereich der Fixierungsbänder reichen. Sie sind unten verschlossen und dienen zur Einführung einer Korrekturhilfe 34 im Falle einer nachträglich erforderlichen Absenkung der Prothese durch operativen Eingriff. Die Operationsstelle kann sich auf den Fixierungsbereich der Fixierbänder 27 beschränken. Das Absenken der Elevationskammer 20 erfolgt von der Operationsstelle aus durch Niederdrücken der Elevationskammer 20 mit Hilfe von Schiebedrähten 34, die von oben in die Führungsrohre 33 eingeführt und nach Absenkung wieder herausgezogen werden.

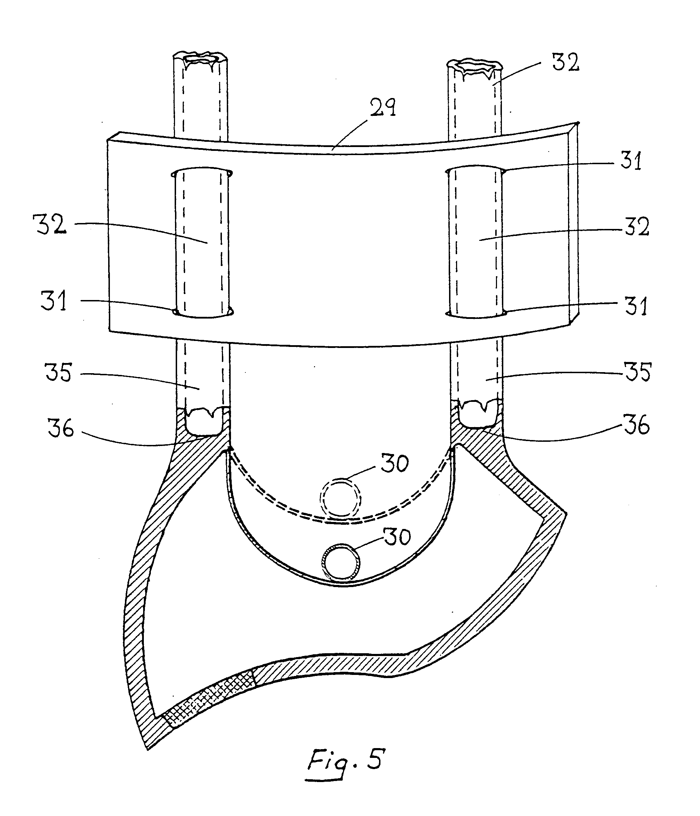

Um besonders bei korpulenten Frauen eine Drehung der Blase bei Streß zu verhindern, kann der erfindungsgemäßen Prothese eine Stützplatte 29 zugeordnet sein, wie dies schematisch in Fig. 5 dargestellt ist. Die Stützplatte 29 kommt bei der Implantation der Prothese in vertikaler Lage vor die Blase und zwar oberhalb der Harnröhre 30. Die Platte besteht aus Kunststoff und kann eben ausgebildet sein. Sie ist vorzugsweise leicht gewölbt in Anpassung an die Form der Blase. Zu ihrer Fixierung weist die Platte vorzugsweise Löcher 31 auf, durch die die Fixierbänder 32 der Prothese geführt sind. Es sind vorzugsweise mindestens vier Löcher 31 vorhanden, wobei ihre Anordnung variieren kann, so daß unterschiedliche Möglichkeiten bestehen, die Fixierbänder durch die Löcher der Platte zu führen, beispielsweise geradlinig, gewunden, über Kreuz oder asymmetrisch.

Die Fixierbänder 32 sind schlauchförmig ausgebildet und innen hohl, wobei der Schlauchraum 35 im Bereich der Elevationskammer verschlossen ist bzw. blind endet, so daß keine Kommunikation mit dem Innenraum der Elevationskammer besteht. Bei einer etwaigen operativen Korrektur kann von oben her ein Draht oder Stab in die schlauchförmigen Fixierbänder eingeführt werden, der an der Kammerwandung 36 zur Anlage kommt, so daß sie nach unten verschoben werden kann.

## Patentansprüche

1. Prothese zur Verhinderung der Harninkontinenz bei Frauen durch Korrektur des vesico-urethralen Winkels mit einer als einstellbarer Abstandhalter zwioschen Vagina und Harnröhrenansatz einsetzbaren, mit Fluid gefüllten bzw. füllbaren, in ihrer Höhe verstellbaren Elevationskammer (1; 20), deren obere Außenfläche (7; 25) als nicht einengende elastische Auflagefläche für die Harnröhre (9; 23) und deren untere Außenfläche (8; 24) als Abstützfläche zum Aufsetzen auf die Oberseite der Vagina ausgebildet sind und deren den Abstand bestimmende Höhe in implantiertem Zustand durch Punktion und Korrektur der Füllmenge einstellbar ist, dadurch gekennzeichnet, daß sie mindestens zwei an der Elevationskammer (1; 20) angeordnete flexible Bänder (2, 3; 27, 28) zur spannungsfreien Fixierung der Lage der Prothese im Körper aufweist.

2. Prothese nach Anspruch 1, dadurch gekennzeichnet, daß die Fixierungsbänder (2, 3; 27) länger ausgebildet sind als erforderlich und auf die richtige Länge kürzbar sind.

3. Prothese nach Anspruch 1 oder 2, dadurch gekennzeichnet, daß die Fixierungsbänder (2, 3; 27) aus dem gleichen gummielastischen Material bestehen wie die Elevationskammer (1; 20), vorzugsweise einstückig mit dieser ausgebildet sind.

4. Prothese nach einem der vorhergehenden Ansprüche, dadurch gekennzeichnet, daß mindestens eine Punktionsstelle (17, 18; 22) vorgesehen ist, die außermittig an der Elevationskammer (1; 20) angeordnet ist und vorzugsweise Teil einer Punktionskammer (4; 19; 21) ist.

5. Prothese nach einem der vorhergehenden Ansprüche, dadurch gekennzeichnet, daß verschiedene Punktionsstellen (17, 18), insbesondere Punktionskammern (4; 19) vorgesehen sind, von denen vorzugsweise mindestens eine (19; 21) an der Elevationskammer (1; 20) angeordnet ist.

6. Prothese nach einem der vorhergehenden Ansprüche, dadurch gekennzeichnet, daß mindestens eine Punktionskammer (19; 21) als seitliche Nebenkammer an der Elevationskammer (1; 20) ausgebildet ist.

7. Prothese nach einem der vorhergehenden Ansprüche, dadurch gekennzeichnet, daß Punktionsstellen (18; 22) von Punktionskammern (19; 21), die im örtlichen Bereich der Elevationskammer (1; 20) vorgesehen sind, eine Einstichrichtung vorgeben, die von der Harnröhre (9; 23) abweist.

8. Prothese nach einem der vorhergehenden Ansprüche, dadurch gekennzeichnet, daß die Elevationskammer (1) mindestens eine mit ihr kommunizierende, räumlich getrennte Teilkammer (4) aufweist, die als Punktionskammer ausgebildet ist.

9. Prothese nach einem der vorhergehenden Ansprüche, dadurch gekennzeichnet, daß mindestens eine Punktionskammer (4) über eine flexible Leitung (5) mit der Elevationskammer (1) verbunden und an für die Punktierung geeigneten Stellen des Körpers implantierbar ausgebildet ist.

10. Prothese nach einem der vorhergehenden Ansprüche, dadurch gekennzeichnet, daß mindestens eine externe Punktionskammer (4) zur Befestigung mindestens eines Fixierbandes (3) ausgebildet ist.

11. Prothese nach einem der vorhergehenden Ansprüche, dadurch gekennzeichnet, daß die obere Auflagefläche (7; 25) aus dünnem, weichelastischem Material besteht und der Höhenabstand der Prothese dadurch veränderbar ist, daß der von der Auflagefläche (7; 25) gebildete Abschnitt in Abhängigkeit vom Füllgrad der Elevationskammer (1; 20) in der Höhe variiert.

12. Prothese nach einem der vorhergehenden Ansprüche, dadurch gekennzeichnet, daß die untere, auf die Vagina aufsetzbare Abstützfläche (8; 24), insbesondere sämtliche Oberflächenabschnitte der Elevationskammer (1; 20), aus gummielastischem Material bestehen und mit Ausnahme des Auflageflächenabschnittes (7; 25) für die Harnröhre (9; 23) im wesentlichen nicht dehnbar sind.

13. Prothese nach einem der vorhergehenden Ansprüche, dadurch gekennzeichnet, daß die Auflagefläche (7; 25) für die Harnröhre (9; 23) in quer zur Harnröhre verlaufender Richtung konkav und in Richtung der Harnröhre vorzugsweise konvex ausgebildet ist.

14. Prothese nach einem der vorhergehenden Ansprüche, dadurch gekennzeichnet, daß ihr eine Stützeinrichtung, insbesondere eine Stützplatte (29) für die Harnblase zugeordnet ist.

15. Prothese nach Anspruch 14, dadurch gekennzeichnet, daß die Stützplatte (29) zu ihrer Halterung durch die Fixierbänder (32) ausgebildet ist und hierzu vorzugsweise Durchbrechungen (31) aufweist, durch die die Fixierbänder (32) hindurchführ sind.

16. Prothese nach einem der vorhergehenden Ansprüche, dadurch gekennzeichnet, daß bis in den Bereich der Elevationskammer (20) reichende, insbesondere an den Fixierbändern (27; 32) angeordnete, Führungseinrichtungen (33) für Korrekturhilfen (34) vorgesehen sind, insbesondere die Fixierungsbänder (32) rohr-oder schlauchförmig ausgebildet oder ihnen Führungsrohre (33) zugeordnet sind.
